Europäisches Patentamt

European Patent Office

Office européen des brevets

(19)

(11) **EP 1 383 512 B1**

(12) **EUROPEAN PATENT SPECIFICATION**

(45) Date of publication and mention
of the grant of the patent:
**08.09.2004 Bulletin 2004/37**

(51) Int Cl.⁷: **A61K 31/70**, A61P 15/02

(21) Application number: **01932070.4**

(22) Date of filing: **03.05.2001**

(86) International application number:
**PCT/IT2001/000212**

(87) International publication number:
**WO 2002/089818 (14.11.2002 Gazette 2002/46)**

(54) **USE OF SUCRALFATE FOR THE TREATMENT OF CERVICAL EROSION**

SUCRALFAT ENTHALTENDES ARZNEIMITTEL ZUR BEHANDLUNG VON ZERVIKALE EROSION

UTILISATION DE SUCRALFATE POUR TRAITER L'EROSION CERVICALE

(84) Designated Contracting States:
**AT BE CH CY DE DK ES FI FR GB GR IE IT LI LU
MC NL PT SE TR**
Designated Extension States:
**AL MK RO SI**

(43) Date of publication of application:
**28.01.2004 Bulletin 2004/05**

(73) Proprietor: **FARMIGEA S.P.A.**
**56121 Pisa (IT)**

(72) Inventors:
• **GORI, Gianfranco**
**P.le Solieri, 1, I-47100 Forli' (IT)**
• **GUIDI, Aditeo**
**P.le Solieri, 1, I-47100 Forli' (IT)**
• **FEDERIGHI, Alberto, c/o Farmigea S.p.A.**
**I-56127 Pisa (IT)**
• **BIANCHINI, Pietro, c/o Farmigea S.p.a.**
**I-56127 Pisa (IT)**
• **BOLDRINI, Enrico, c/o Farmigea S.p.a.**
**I-56127 Pisa (IT)**

(74) Representative: **Banchetti, Marina et al**
**c/o Ing. Barzanò & Zanardo Roma S.p.A.**
**Via Piemonte, 26**
**00187 Roma (IT)**

(56) References cited:
**WO-A-99/43333** **US-A- 5 945 446**

Remarks:
The file contains technical information submitted
after the application was filed and not included in this
specification

EP 1 383 512 B1

**Description**

The present invention concerns the use of sucralfate for the treatment of cervical erosion. More specifically, the invention concerns the use of the hydrous basic aluminium salt of sucrose octasulfate known as sucralfate, or of the corresponding non-complexed sulfate salt (i.e. sucrose octasulfate), for the prophylaxis and treatment, by topical intravaginal administration, of the affection of the uterine cervix known as cervical erosion.

As it is known, cervicitis is a widespread acute or chronic inflammation of the uterine cervix which normally represents a complex problem from the etiopathogenetic point of view. This because such affection normally results from a series of coexisting conditions, such as the presence of an ectopic columnar epithelium (i.e., an abnormally placed portion of columnar epithelium, which in normal conditions lines the upper portion of the cervical canal), the occurrence of a cervical hypertrophy and the presence of not fully resolved old lacerations.

In general, acute cervicitis is an infected and inflamed state of the cervix marked by redness, oedema and bleeding on contact, the symptoms of which do not always occur but may include foul-smelling discharge from the vagina, pelvic pressure or pain, scant bleeding with intercourse and itching or burning of the external genitalia. An inflammatory acute cervicitis may directly develop to affect the uterine cervix or it may be secondary to a vaginal or uterine infection. Also, the infection may be primary or it may aggravate a pre-existing mechanical or chemical lesion. The causative agents are mainly bacteria and other microorganisms, and when the phlogosis is sustained by a microbial combination it may be quite difficult to identify the causative germs, even upon culture examination. Specially in the most industrially developed countries, as a consequence of the growing use of oral contraceptives the most frequent cause of cervical infections is *Candida albicans,* while *Trichomonas vaginalis* is the second cause. *Corinebacterium vaginalis* is often responsible for the occurrence of vaginitis, which secondarily affects the cervical mucosa. Further, *Neisseria gonorrheae* is a frequent causative agent of acute cervicitis and it results in a direct infection of the cervical mucous membrane by the gonococci. Although the non-specific antibiotics generally destroy gonococci, invasive microorganisms may persist for months or even years, and may give rise to chronic cervicites. In addition, non-specific infections may occur together with the main infection, and they may result in lacerations of the uterine cervix.

Also viruses may infect the cervix. In particular, *Herpes simplex,* most frequently of type 2, results in vesicle-shaped transitory lesions of the mucosal surface, which lesions may often become ulcerated.

As a frequent occurrence, the uterine cervix affected by an inflammatory process may show, besides the signs of the developing phlogosis, some aspects which may be ascribed to pre-existing chronic lesions, re-acutized by the ongoing process. In turn, the pre-existing lesions may render the ongoing inflammatory process a chronic one. In general, chronic cervicitis is a persistent inflammation of the cervix that usually occurs among women in their reproductive years. Symptoms include a thick, irritating, malodorous discharge that may in severe cases be accompanied by pelvic pain. The cervix looks congested and enlarged and there are signs of eversion of the cervix and often old lacerations from childbirth.

The above acute and chronic conditions of the uterine cervix are often accompanied by cervical ectopias, i. e. abnormally placed zones of columnar epithelium, which is the mucus-producing tissue lining the internal uterine canal, onto the external part of the cervix, the latter being normally covered by squamous epithelium. The occurrence of cervical ectopias is a quite frequent situation, specially in multiparous women and in those who have had early sexual activity.

In a normal hystological picture, the morphologic examination of the external portion of the intravaginal section of the uterus neck (referred to as "*portio*") shows it to be smooth and lined by a mucous surface similar to the one lining the vagina. Such mucous surface consists of a squamous multilayered epithelium, made of glycogen-containing cells. The passage from the multi-layer squamous epithelium of the cervix *portio* to the mucus-producing columnar epithelium of the cervix canal is sharp. Normally, the borderline is placed immediately behind the external os of the cervix.

As a frequent occurrence specially in women who have undergone childbirth, small islands of columnar epithelium cells are displaced from the cervical canal to various positions onto the external *portio*, thereby resulting in the so-called cervical erosion. Thus, the latter is a condition in which the squamous epithelium of the cervix is abraded as a result of irritation caused by infection or trauma, such as childbirth, and replaced by columnar epithelium. The areas of cervical erosion become easily inflamed and are often responsible for an increased intermenstrual vaginal discharge (leucorrhea). Most importantly, early treatment of cervical erosion is desirable to prevent possible malignancy. Actually, if not suitably treated, cervical erosion may give rise to cancer of the uterine cervix, also known as cervical carcinoma, which is considered to be the cause of about 10% of the deaths from cancer in the woman.

In view of the foregoing, cervical erosion is a condition which calls for careful consideration particularly if it occurs in a situation of cervical and/or vaginal inflammation. In a clinical study on 118 patients who had undergone colposcopic examination (i.e., an examination of the vagina and cervix with an optical magnifying instrument) with abnormal findings, carried out by Frank Gerard (Gerard, F., Topography of abnormal colposcopy findings, The Cervix, 11, 45-52, 1993) it was shown that cervical erosion accounted for 5% of the overall abnormal findings.

The usual treatment of cervical erosion consists of surgery, generally by cauterization, electrocoagulation or cryosurgery, since no topical or systemic pharmacological treatment has been found to date. Cauterization of the cervix or dilatation of the same, which may be carried out on a day-hospital basis, cannot be performed in the pre-menstrual phase, in view of the risk of post-surgical infections. If the cervical canal shows any lacerations or a serious chronic cervicitis other interventions should be performed, such as, in particular, electrocoagulation. The latter allows to closely control the rate of heat penetration and of tissue destruction. Cryosurgery destroys the tissue by freezing it, with the advantage that bleeding after surgery and cervical stenosis have a reduced occurrence.

Before performing one of the above surgical interventions, however, it is necessary to consider the adverse effects that may result from the surgery, i.e. the possibility of hemorrhages or infections after surgery, the possibility of stenosis formation, as well as the risk of infertility and possible dystocia (i.e., pathologic or difficult labor) in case of future pregnancy. In all cases, the result of the adoption of one of the above surgical methods is the destruction of the affected tissue, with subsequent healing by fibroblast proliferation and re-epithelization. Another drawback of the surgical intervention is the increase of the leuchorrea discharge for about two-three weeks. In addition, the cauterization may result in the reactivation of a salpingitis if the treatment is carried out in the course of an acute cervicitis; cervical stenosis may result as a consequence of a deep cauterization; finally, cauterization and cryosurgery carried out on a cervix wherein an undetected neoplasm is present may hide the cancerous process thereby delaying the diagnosis, with the risk of serious consequences.

The evaluation of the recovery of a cervical erosion is normally based on the direct colposcopic examination of the interested area. In this connection, it is reasonable to consider that any abnormal morphology observed in the mucous membrane after healing may negatively affect the protection of said membrane from the recurrence of cervical erosion, when ulcerogenic factors are present. Therefore, a regular morphology of the cervical mucous membrane in the previously eroded area is a critical factor in avoiding the recurrence of the lesions.

A particularly important factor which positively contributes to the remodeling of the mucous membrane tissue and to the restoration of the normal function of said membrane, as well as of the mechanical resistance thereof, is the fibrinolysis enzymatic system, which is present in the cervical mucus. A tissue activator of the fibrinolytic system has been first detected both in the myometrium and in the endometrium. The menstrual fluid contains plasmin and an activator which is biochemically comparable to the tissue activator detected in the endometrium. In addition, both plasminogen and fibrinogen are present in small amounts in the menstrual fluid. Some authors studied the fibrinolysis enzymatic system through the menstrual cycle, by taking samples of cervical mucus from women with ovulatory cycles at various stages of the cycle and analyzing such samples for the presence of the components of the fibrinolytic system. It turned out that an enzymatic activator present in the cervical mucus fell at a low level two days before the ovulation, remained at such low level during and right after the ovulation and then increased in the following days. Acceptable amounts of plasmin and of pro-activator and, to a lower extent, of plasminogen were detectable.

The above observations allowed to ascertain that a fibrinolytic system is active in the cervical mucus produced by the columnar cells of the cervical epithelium. As it is known that the fibrinolysis enzymatic system performs a primary function in the restoration of the normal architecture of cicatricial tissue cells, thus contributing in a critical manner to the restoration quality of the cervical lesion, it is an object of the present invention to supply directly to the eroded cervical mucous membrane a biologically active agent which may promote a satisfactory healing of the cervical erosion, as an alternative to surgical treatment. The proposed agent should exert a protective activity against the damage caused by possible inflammatory factors which may be released *in situ* and, most importantly, should be able to induce a "*restitutio ad integrum"* of the affected tissue, which goal is not achievable through the surgical treatment or through any known therapeutic approach.

In particular, the proposed pharmaceutical agent should be active in preserving and maintaining *in situ* the fibrinolytic system which is present in the cervical mucus, in order that said system may perform its fundamental role in the process of remodeling the cellular architecture of the damaged area.

Within the frame of the studies on the protein components of cervical mucus it has been found that sucralfate, a basic aluminium sucrose sulfate complex which is known to inhibit, upon oral administration, peptic hydrolysis and stomach acidity, shows a remarkable activity, upon topical application on the cervical erosion site, in preserving *in situ* the factors of the fibrinolytic system and in making such factors available to perform their function in the dynamic formation of the cellular layer which integrally restores the eroded tissue of the cervical mucous membrane.

Sucralfate is an amorphous complex of sulfated sucrose and aluminium hydroxide (specifically, $\beta$-D-fructofuranosyl-$\alpha$-D-glucopyranoside octakis(hydrogen sulfate) aluminium complex) currently used as a cytoprotective agent in the management of peptic ulcer and chronic gastritis. Under acid gastro-intestinal conditions this agent forms a polymeric gel provided with a number of different protective properties. The compound releases aluminium ions, thus acquiring a strong negative charge through which it electrostatically binds to pepsin and other mucins (mucus glycoproteins). The concentration of pepsin A in the gastric lumen is thus reduced and the pepsin-substrate bond is inhibited by competition. The $Na^+/H^+$ exchange in the mucus is increased, thereby increasing the neutrality gradient on the mucous membrane, and the degradation of mucins from active pepsin A (at pH > 5) is inhibited.

Sucralfate is reported to have a special affinity for ulcer sites. By binding with mucins it forms a gel protecting the peptic lesion, which is also believed to activate trophic and healing processes. The latter are ascribed partly to the stimulation of the arachidonic acid metabolism and to the production of the $PGE_1$ and $PGE_2$ prostaglandins, and partly to the increased presence of epidermal growth factor (EGF) and transforming growth factor $\alpha$ (TGF$\alpha$). These factors are known to stimulate the ulcer reparation mechanisms.

In duodenal ulcer sucralfate is reported to have a clinical effectiveness in 75% of cases with the oral dose of 1 g four times daily, for 4-8 weeks. In gastric ulcer, the ulcer site plays a major role in the effectiveness of sulcralfate administration, the effectiveness being highest in the gastric body ulcers and in the pre-pyloric ulcers (after 6-8 weeks of treatment). In NSAID-induced ulceration (ulcers induced by the administration of non-steroidal antiinflammatory drugs) sucralfate is effective in duodenal localizations, but has only a symptomatic and non-reparative effect when gastric lesions are present. The oral administration of sucralfate is also indicated in stress-induced ulceration, while the topical oral administration of sucralfate is indicated in the prophylaxis and treatment of mouth ulceration, such as stomatitis induced by cancer therapy.

Besides the conventional use in the treatment of gastrointestinal ulcer and gastritis, as well as in the treatment of mouth ulceration, sucralfate and related sulfated saccharides have been disclosed, in the co-filed international patent applications WO 89/05645 and WO 89/05646, as being active by topical application to the skin or to mucosal surfaces for the prophylaxis or treatment of inflammation and/or infection. According to the two disclosures, sucralfate and, in general, sulfated mono- or disaccharides and salts and complexes thereof, are claimed to be effective against any type of inflammatory disease irrespective of the cause thereof, namely caused by, e.g., a microbial epithelial infection, a non-microbial dermatosis, an allergic or immune disorder, a malignant or premalignant disease, exposure to radiation, to a chemical agent and so on. Among the possible applications in the gynecologic field, WO 89/05645 and WO 89/05646 recite the treatment of inflammation and vaginosis caused by microbial and viral infections, such as herpes simplex, infection by mycoplasma, chlamidia, candida, trichomonas, etc., and the treatment and prophylaxis of inflammation caused by cervical dysplasia and carcinoma. As the wide range of possible therapeutic uses is focused on the treatment of the inflammatory symptom irrespective of the causative agent thereof, the above disclosure is not concerned with the treatment of cervical erosion, which is an affection that may occur independently of a possible concurrent inflammatory process.

According to the present invention, as pointed out in the foregoing, it has been found that sucralfate, formulated in topical preparations for intravaginal administration, may be effectively employed for the treatment of cervical erosion. This specific therapeutic activity is due to the fact that sucralfate, by preserving *in situ* the action that the fibrinolytic enzymatic system performs in the cell reparation process, favors a complete *restitutio ad integrum* of the cervical mucous epithelium without the appearance of any cicatricial signs.

Accordingly, the present invention specifically provides the use of sucralfate, i.e. $\beta$-D-fructofuranosyl-$\alpha$-D-glucopyranoside octakis(hydrogen sulfate) aluminium complex, or of its component sucrose octasulfate (SOS) for the preparation of a topical intravaginal medicament for the restoration of abraded epithelial tissue of the cervical mucous membrane occurring in cervical erosion. Said topical intravaginal medicament preferably contains, according to the invention, from 5% to 30% by weight of sucralfate or of the corresponding sucrose octasulfate.

According to some preferred embodiments of the invention, the proposed intravaginal medicament contains one or more bioadhesive or mucoadhesive carriers, such as, e.g., hydroxypropylcellulose, carbomers, alginates, pectin, xyloglucanes, so as to obtain an increased *in situ* residence time of the active ingredient, in the presence of a high concentration gradient. In a particularly preferred embodiment, sucralfate is carried by a bioadhesive gel based on polycarbophil, which promotes the constant and gradual release of the active ingredient in the action site. After the administration on the area affected by the cervical erosion, the preparation containing polycarbophil as the bioadhesive vehicle adheres to the mucln-epithelial surface for a prolonged period of time. This affords, in addition, a homogeneous diffusion of the drug on the damaged cervical mucosa with no loss of product, contrary to what may happen with the conventional vaginal preparations lacking a bloadhesive ingredient. Preferably, said topical intravaginal medicament is in the form of a gel containing from 10% to 30% by weight of sucralfate and polycarbophil as a bioadhesive/mucoadhesive ingredient.

When using a preparation containing from 10% to 30% by weight of sucralfate in a bioadhesive carrier based on polycarbophil, the preparation may be applied at a dosage of 2 g once daily for 6-8 days in order to obtain a complete restoration of the eroded area of the uterine cervix. In general, the dosage and the posology may undergo variations with no detrimental effect on the prevention and maintenance action on the enzymatic system (glycocalyx) or on the mucus thus produced.

According to some further preferred embodiments of the invention, besides the version in the gel form, the preparation may be produced in any one of the known pharmaceutical forms suitable for vaginal administration, such as, e.g., in the form of a cream, a paste, an emulsion, an ointment, a vaginal suppository, a solution or a suspension. Preferably, in order to achieve a good tolerability and a satisfactory maintenance of the vaginal ecosystem, the optimal pH of the medicament should be comprised between 4.0 and 5.5.

Still according to the invention, in addition to the bioadhesive and mucoadhesive carriers the preparation may contain further optional ingredients, such as thickening agents, antioxidants, stabilizers, surfactants, etc.. Further, the preparation may contain preservatives and antimicrobial agents, such as methyl-, ethyl- and propyl-paraben, benzoic acid, benzyl alcohol.

According to another aspect of the invention, there is provided a method for the in situ application of a medicament as described in the foregoing, which method includes the use of an intravaginal cannula, provided with a suitable applicator means. This delivery system allows a close contact of the active ingredient with the mucosa of the uterine cervix, where the drug is required to perform its therapeutic action.

Some specific embodiments of sucralfate preparations in gel, suitable for the application according to the invention, are described below for merely illustrative purposes, together with the results of the clinical studies carried out on the proposed agent, in order to test its effectiveness for the medical indication proposed.

EXAMPLE 1

Gel preparation for topical intravaginal application — Formulation 1

A gel having the following formulation was prepared according to the procedure described below.

| micronized sucralfate | g 12.50 |
|---|---|
| polycarbophil (Noveon AA1) | g 0.85 |
| methyl-paraben | g 0.20 |
| propyl-paraben | g 0.02 |
| triethanolamine | g 0.39 |
| propylene glycol | g 86.04 |
| | g 100.00 |

Preparation procedure :

In a suitable mixer provided with a heat exchange system and a stirrer, g 0.85 g of polycarbophil, g 0.20 of methyl-paraben and 0.02 di propyl-paraben are added to g 86.04 of propylene glycol. The suspension is heated to 70°C under continuous stirring until complete dissolution of the ingredients. The thus obtained solution is allowed to cool to 50°C and at this temperature g 12.50 of micronized sucralfate is added. The stirring is continued also using a turbine for some minutes (2-3 treatments with the turbine) until complete cooling is reached. Finally, triethanolamine is added while using the turbine, until a translucid white gel is obtained, having a good appearance.

The pH of the gel thus produced is 4.8.

In order to measure the pH, 50 ml of deionized water are added under stirring to 10.0 g of gel. The mixture is then stirred and pH is determined on the dispersion thus obtained.

EXAMPLE 2

Gel preparation for topical intravaginal application - Formulation 2

A gel similar to the previous one but having a different proportion of the ingredients was prepared according to the same procedure described in Example 1. The formulation was as follows.

| micronized sucralfate | g 25.00 |
|---|---|
| polycarbophil (Noveon AA1) | g 1.70 |
| methyl-paraben | g 0.20 |
| propyl-paraben | g 0.02 |
| triethanolamine | g 0.40 |
| propylene glycol | g 72.68 |
| | g 100.00 |
| pH = 4.7 | |

EXAMPLE 3

Gel preparation for topical intravaginal application - Formulation 3

A gel similar to the previous ones but having a different proportion of the ingredients was prepared according to the same procedure described in Example 1. The formulation was as follows.

| | |
|---|---|
| micronized sucralfate | g 12.50 |
| polycarbophil (Noveon AA1) | g 1.70 |
| methyl-paraben | g 0.20 |
| propyl-paraben | g 0.02 |
| triethanolamine | g 0.40 |
| propylene glycol | g 85.18 |
| | g 100.00 |
| pH = 4.9 | |

EXAMPLE 4

Gel preparation for topical intravaginal application - Formulation 4

A further gel similar to the previous ones but having a different proportion of the ingredients was prepared according to the same procedure described in Example 1. The formulation was as follows.

| | |
|---|---|
| micronized sucralfate | g 10.00 |
| polycarbophil (Noveon AA1) | g 3.00 |
| methyl-paraben | g 0.20 |
| propyl-paraben | g 0.02 |
| triethanolamine | g 0.40 |
| propylene glycol | g 86.78 |
| | g 100.00 |
| pH = 4.5 | |

Experimental results

The results of some clinical trials aimed at testing the effectiveness of the use of sucralfate proposed according to the invention are reported below.
The test has been carried out on 18 women, aged between 18 and 46, who upon colposcopic analysis showed various degrees of cervical erosion.
Based on the observed colposcopic picture of the situation and on the seriousness of the possible accompanying inflammation state the patients under test have been classified according to a conventional classification of the cervical pathology, according to the following grade scheme.

- *Grade I: simple cervicitis; presence of mosaic-shaped lesions and ectopia; reduced leucorrhea and absence of dyspareunia (abnormal pain during sexual intercourse);*
- *Grade II: cervicitis with extended erosion of the portio (30% of the surface); presence of altered epithelium; occasional blood discharge and occasional dyspareunia;*
- *Grade III: cervicitis with extended erosion of the portio (50% of the surface); presence of ectopia; frequent blood discharge; leucorrhea and dyspareunia.*

Thus, the overall situation of the patients before starting the pharmacological treatment was as summarized in the following table.

TABLE 1

| Clinical tests on topical sucralfate for the treatment of cervical erosion Baseline evaluation of the patients | | | | |
|---|---|---|---|---|
| Patient no. | Age | No. of child-births | Ongoing therapy | Cervical pathology grade |
| 1 | 24 | 0 | None | I |
| 2 | 20 | 0 | None | I |
| 3 | 36 | 1 | Oral contraceptive | II |
| 4 | 38 | 2 | None | I |
| 5 | 45 | 2 | None | I |
| 6 | 34 | 0 | Oral contraceptive | I |
| 7 | 28 | 1 | None | I |
| 8 | 19 | 0 | Oral contraceptive | II |
| 9 | 23 | 1 | None | II |
| 10 | 20 | 0 | Oral contraceptive | I |
| 11 | 22 | 1 | Oral contraceptive | I |
| 12 | 46 | 2 | None | I |
| 13 | 32 | 1 | None | II |
| 14 | 27 | 1 | Oral contraceptive | III |
| 15 | 40 | 0 | Antibiotics | III |
| 16 | 42 | 1 | AZT*+ oral contraceptive | III |
| 17 | 40 | 1 | None | I |
| 18 | 31 | 1 | Oral contraceptive | I |

* AZT = zidovudine

The 18 women, after final diagnosis, were divided into two test groups of nine patients each, in order to evaluate the effectiveness of the treatment according to the invention in two different formulations. The first formulation, containing sucralfate at a concentration of 25% by weight as well as polycarbophil as a mucoadhesive/bioadhesive ingredient, corresponds to the preparation referred to above as Formulation 2, while the second formulation under test, containing sucralfate at a concentration of 12.5% by weight as well as polycarbophil, corresponds to the Formulation 3.

Each one of the selected patients received 2 g of gel, by direct application onto the uterine cervix, once daily at night for six consecutive days.

The overall evaluation of the effectiveness of the preparations under test, at the end of the topical treatment, is reported in the two following tables.

TABLE 2

| Colposcopic examination results and evaluation of signs and symptoms Formulation 2 (sucralfate 25%) | | | |
|---|---|---|---|
| Patient no. | Cervical pathology grade | Results | Side effects |
| 10 | I | Complete restoration and restitutio ad integrum of the cervical epithelium | None |
| 11 | I | do. | None |
| 12 | I | do. | None |
| 13 | II | do. | None |
| 14 | III | do. | None |
| 15 | III | do. | None |

TABLE 2   (continued)

| Colposcopic examination results and evaluation of signs and symptoms Formulation 2 (sucralfate 25%) | | | |
|---|---|---|---|
| Patient no. | Cervical pathology grade | Results | Side effects |
| 16 | III | do. | None |
| 17 | I | do. | None |
| 18 | I | do. | None |

TABLE 3

| Colposcopic examination results and evaluation of signs and symptoms Formulation 3 (sucralfate 12.5%) | | | |
|---|---|---|---|
| Patient no. | Cervical pathology grade | Results | Side effects |
| 1 | I | Complete restoration and restitutio ad integrum of the cervical epithelium | None |
| 2 | I | do. | None |
| 3 | II | do. | None |
| 4 | I | do. | None |
| 5 | I | do. | None |
| 6 | I | do. | None |
| 7 | I | do. | None |
| 8 | II | Restoration with redness in about 20% of the *portio* surface | None |
| 9 | II | do. (patient no. 8) | None |

As it may be observed from the preceding data, 16 patients out of 18 could be considered on complete recovery after six weeks of treatment with sucralfate. Two patients, who had been treated with the gel at the lowest concentration (12.5%) appear to need a longer treatment, with respect to the fixed six days period, in order to achieve a full restoration of the normal epithelium of the cervical mucous membrane.

The present invention has been disclosed with particular reference to some specific embodiments thereof, but it should be understood that modifications and changes may be made by the persons skilled in the art without departing from the scope of the invention as defined in the appended claims.

**Claims**

1.   Use of sucralfate, i.e. β-D-fructofuranosyl-α-D-glucopyranoside octakis(hydrogen sulfate) aluminium complex, or of its component sucrose octasulfate for the preparation of a topical intravaginal medicament for the restoration of abraded epithelial tissue of the cervical mucous membrane occurring in cervical erosion.

2.   The use according to claim 1, wherein said topical intravaginal medicament contains from 5% to 30% by weight of sucralfate or of the corresponding sucrose octasulfate.

3.   The use according to claims 1 or 2, wherein said topical intravaginal medicament contains from 10% to 30% by weight of sucralfate.

4.   The use according to any one of claims 1-3, wherein said topical intravaginal medicament also contains one or more bioadhesive or mucoadhesive carriers.

5.   The use according to claim 4, wherein said bioadhesive or mucoadhesive carrier is selected from the group consisting of hydroxypropylcellulose, carbomers, alginates, pectin, xyloglucanes and polycarbophil.

6. The use according to any one of claims 1-5, wherein said topical intravaginal medicament is in the form of a gel containing from 10% to 30% by weight of sucralfate and polycarbophil as a bioadhesive/mucoadhesive ingredient.

7. The use according to any one of claims 1-5, wherein said topical intravaginal medicament is in the form of a cream, a paste, an emulsion, an ointment, a vaginal suppository, a solution or a suspension.

8. The use according to any one of claims 1-7, wherein said topical intravaginal medicament has a pH comprised between 4.0 and 5.5.

9. The use according to any one of claims 1-5, wherein said topical intravaginal medicament is suitable to be administered on the affected site by means of an intravaginal cannula, provided with a suitable applicator means.

**Patentansprüche**

1. Einsatz von Sucralfat, das heißt β-D-fructofuranosyl-α-D-Glukopyranoside octakem (hydrogen sulfat) Aluminiumkomplex, oder von dessen Bestandteil Sucrose-Oktasulfat zur Vorbereitung eines lokalen intrevaginalen Medikaments zur Wiederherstellung von abgeschürftem Epithelgewebe der Zervixschleimhaut, wie sie bei Zervixschwund vorkommt.

2. Einsatz gemäß Anspruch 1, **dadurch gekennzeichnet, dass** das besagte lokale intravaginale Medikament zwischen 5 und 30 Gewichtsprozent Sucralfat oder entsprechendes Sucrose- Oktasultat beinhaltet.

3. Einsatz gemäß Anspruch 1 oder 2, **dadurch gekennzeichnet, dass** das besagte lokale intravaginale Medikament zwischen 10 und 30 Gewichtsprozent Sucralfat beinhaltet.

4. Einsatz gemäß einem der Ansprüche 1 bis 3, **dadurch gekennzeichnet, dass** das besagte lokale intravaginale Medikament außerdem einen oder mehrere bioadhäsive oder mucoadhäsive Träger beinhaltet.

5. Einsatz gemäß Anspruch 4, **dadurch gekennzeichnet, dass** der besagte bioadhäsive oder mucoadhäsive Träger aus einer Gruppe ausgewählt ist, bestehend aus Hydroxypropylzellulose, Carbomer, Alginat, Pektin, Xyloglukan und Polycarbophil.

6. Einsatz gemäß einem der Ansprüche 1 bis 5, **dadurch gekennzeichnet, dass** das lokale intravaginale Medikament die Form eines Gels hat, das 10 bis 30 Gewichtsprozent Sucralfat und Polycarbophil als einen bioadhäsiven / mucoadhäslven Bestandteil umfasst.

7. Einsatz gemäß einem der Ansprüche 1 bis 5, **dadurch gekennzeichnet, dass** das lokale intravaginale Medikament die Form einer Creme, einer Paste, einer Emulsion, einer Salbe, einem Vaginatsuppositorium, einer Lösung oder einer Suspension hat.

8. Einsatz gemäß einem der Ansprüche 1 bis 7, **dadurch gekennzeichnet, dass** das lokale intravaginale Medikament einen pH-Wert zwischen 4,0 und 5,5 aufweist.

9. Einsatz gemäß einem der Ansprüche 1 bis 5, **dadurch gekennzeichnet, dass** das lokale intravaginale Medikament geeignet ist, an der betroffenen Stelle mittels einer intravaginalen Kanüle, die mit einem geeigneten Applikator versehen ist, verabreicht zu werden.

**Revendications**

1. Utilisation du sucralfate, c'est-à-dire du complexe d'aluminium d'octakis(hydrogénosulfate) de β-D-fructofuranosyl-α-D-glucopyrannoside, ou de son composant octasulfate de saccharose pour la préparation d'un médicament topique intravaginal destiné à la reconstitution du tissu épithélial abrasé de la muqueuse cervicale apparaissant dans une érosion cervicale.

2. Utilisation selon la revendication 1, dans laquelle ledit médicament topique intravaginal contient 5 % à 30 % en poids de sucralfate ou de l'octasulfate de saccharose correspondant.

**3.** Utilisation selon les revendications 1 ou 2, dans laquelle ledit médicament topique intravaginal contient 10 % à 30 % en poids de sucralfate.

**4.** Utilisation selon l'une quelconque des revendications 1 à 3, dans laquelle ledit médicament topique intravaginal contient également un ou plusieurs supports bioadhésifs ou mucoadhésifs.

**5.** Utilisation selon la revendication 4, dans laquelle ledit support bioadhésif ou mucoadhésif est choisi dans le groupe formé par l'hydroxypropylcellulose, les carbomères, les alginates, la pectine, les xyloglucanes et le polycarbophile.

**6.** Utilisation selon l'une quelconque des revendications 1 à 5, dans laquelle ledit médicament topique intravaginal est sous la forme d'un gel contenant 10 % à 30 % en poids de sucralfate et du polycarbophile comme ingrédient bioadhésif/mucoadhésif.

**7.** Utilisation selon l'une quelconque des revendications 1 à 5, dans laquelle ledit médicament topique intravaginal est sous la forme d'une crème, d'une pâte, d'une émulsion, d'une pommade, d'un suppositoire vaginal, d'une solution ou d'une suspension.

**8.** Utilisation selon l'une quelconque des revendications 1 à 7, dans laquelle ledit médicament topique intravaginal a un pH compris entre 4,0 et 5,5.

**9.** Utilisation selon l'une quelconque des revendications 1 à 5, dans laquelle ledit médicament topique intravaginal convient pour être administré au site affecté au moyen d'une canule intravaginale munie d'un moyen applicateur approprié.